# EUROPEAN PATENT SPECIFICATION

(11) **EP 0 635 669 B1**
(45) Date of publication and mention of the grant of the patent: **03.09.1997**
(21) Application number: 94202128.8
(22) Date of filing: 21.07.1994
(51) Int. Cl.: F16L 37/00, H01R 13/629, A61M 39/00

(54) **Connecting device**
Verbindungsvorrichtung
Dispositif de raccordement

(30) Priority: 23.07.1993 NL 9301297
(43) Date of publication of application: 25.01.1995
(73) Proprietor: Portanje Elektronika B.V., Amsterdam (NL)
(72) Inventor: Balk, Annette Hermiene, NL-6401 HL Roermond (NL)
(74) Representative: de Vries, Johannes Hendrik Fokke

(56) References cited:
- DE-A- 1 778 746
- FR-A- 2 546 676
- GB-A- 2 143 295
- US-A- 2 256 780

## Description

The invention relates to a device in accordance with the preamble of appended claim 1.

A device of this kind is known from DE-A-1 943 552. According to this document the connecting box, for example a wall connecting box, is connected with a line system provided in a hospital. The advantage of such a device is that the connecting element is connected with the connecting box in the parking position, however, there is not yet provided a connection between the user and the gas source. By a small movement from the parking position into the working position a closing valve generally accommodated in the connecting box, is opened whereby the user is connected with the gas source. In the known device a problem arises as it cannot be observed well from some distance whether the connecting element is located in the parking position or the working position, whereby undesired situations may occur. The displacement of the connecting element from the working position into the parking position is only approximately 3 mm. A displacement of this small size is not suitable for reliable indication purposes and, moreover, the displacement can only be observed from the side.

The invention aims to provide a simple and efficient solution for this problem.

To this end the device according to the invention is characterized in accordance with the characterizing portion of claim 1.

The invention thus relates to a connecting device which has been provided with a coloured indication means which is three-dimensionally shaped and therefore may be observed from any side to observe the presence of the colour. The device is suitable for use in hospital or operating theatre environments, provides excellent three-dimensional indication, which is directly related to whichever of the working or parking positions is occupied and is mechanically and reliably operated by completely automatic means. Even a small axial displacement results in a well observable indication.

Advantageous embodiments of the invention are defined in the depending claims 2 - 5.

The invention will be further explained by reference to the drawings in which an embodiment is schematically shown.

Fig. 1 is a schematical side view partially shown in cross section of an embodiment of the device according to the invention, wherein the connecting element is located in the parking position.

Fig. 2 is a side view of the device of Fig. 1 partially shown in cross section, wherein the connecting element is located in the working position.

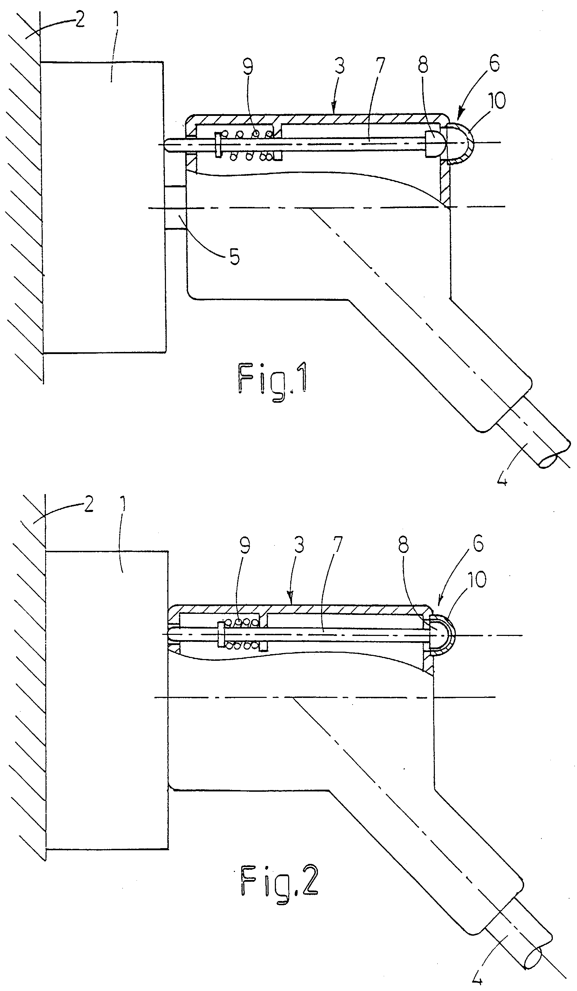

Fig. 1 shows partially in cross section and partially in a side view a device for connecting a user not further shown to a neither shown source of medical gas or a vacuum source. This gas can for example be oxygen, laughing-gas or the like. The device comprises a connecting box 1 which in this case is mounted on a wall 2 of a hospital and is permanently connected to the line system mounted in the wall 2 and leading to a gas source located elsewhere in the hospital. Of course, the connecting box can be adapted to be flush mounted in the wall. The device further comprises a connecting element 3 with a hose 4 leading to the user. The connecting element 3 can be detachably coupled with the connecting box 2 by inserting a nipple 5 in an opening of the connecting box 1 which is not visible. In Fig. 1 the connecting element 3 is coupled in a parking position with the connecting box 1 wherein a closing valve accommodated in the connecting box 1 is still closed so that the user is not yet connected with the gas source. Thereby it is obtained that the connecting element 3 can be brought in a position in the connecting box 1 ready for use without directly connecting the user with the gas source.

In the embodiment shown in the drawing the connecting element 3 can easily be moved from the parking position into a working position by pressing the connecting element in axial direction of the nipple 5 towards the connecting box 1 into the position shown in Fig. 2. By this axial movement the closing valve accommodated in the connecting box 1 is opened so that the user is connected with the gas source.

As the movement of the connecting element 3 from the parking position into the working position is only small, it is difficult to observe from some distance in which position the connecting element 3 is located. In order to overcome this problem the connecting element 3 is provided with an indicating means 6 which in the embodiment shown comprises a pin 7 slidably received in the connecting element 3. This pin 7 projects out of the connecting element 3 at the side of the nipple 5 and is provided with a coloured head 8 at the opposite side. The pin 7 is pressed into the position of Fig. 1 by a spring 9. In this position corresponding with the parking position of the connecting element 3, the head 8 of the pin 7 is not visible in the semispherical window 10 mounted on the side of the connecting element 3 directed away from the nipple 5.

When the connecting element 3 is moved from the parking position into the working position, the head 8 of the pin 7 is pressed into the window 10 so that this head 8 can be well observed through the window 10. By the semispherical shape of the window 10 and the head 8 it is obtained that the head 8 can be observed very well from each direction. Thereby, the staff can ascertain whether the connecting element 3 is located in the parking position or in the working position.

It will be clear that the indicating means 6 can be made in different ways. The indicating means can for example be provided with two differently coloured indicating faces which are visible through a window in the parking position and the working position, respectively. Further, it is also possible to actuate the indicating means by the gas pressure instead of in a mechanical way as described. It is also possible to detect occupying the parking position and the working position in an optical or electronical manner and to provide a visual and/or audible indication through a suitable processing means. Finally, it is noted that the indicating means can also be accommodated in the connecting box 1, if desired.

Although the connecting element is moved from the parking position into the working position perpendicular to the wall 2 in the embodiment described, it is of course also possible to use a movement mainly parallel to the wall.

## Claims

1. Device for connecting a user to a source of medical gas, such as for example oxygen, or a vacuum source, comprising a connecting box (1) which can be permanently connected to the source, and a connecting element (3) which can be detachably coupled with the connecting box and can be permanently connected with the user, wherein the connecting element can be coupled with the connecting box in a parking position and in a working position, wherein the user is connected to the source in the working position only, and wherein an indication means (7, 8, 10) indicates the position of the connecting element (3) in the connection box (1), **characterized** in that the indicating means (7, 8, 10) comprises a window (10) in which a colour indication is visible, which indicating means comprises a pin (7) slidably mounted in the connecting element (3), said pin having a coloured head (8) pressed by a spring (9) away from the window (10) and moved towards the window against the action of the spring during movement from the parking position into the working position, and in that the window (10) is shaped three-dimensionally, the head (8) of the pin (7) lying fully within the three-dimensional window in the working position.

2. Device according to claim 1, **characterized** in that the pin (7) with its end opposite of the head (8) projects out of the connecting element (3) and cooperates with said end with the connecting box (1).

3. Device according to claim 1, **characterized** in that the indication means (7, 8, 10) is provided in the connecting element (3) .

4. Device according to claim 1 or 3, **characterized** in that the indicating means (7, 8, 10) is actuated by the movement of the connecting element (3) from the parking position into the working position.

5. Device according to claim 1 or 2, **characterized** in that the indicating means (7, 8, 10) is actuated by the gas pressure.

## Patentansprüche

1. Vorrichtung zum Verbinden eines Benutzers mit einer Quelle medizinischen Gases, wie zum Beispiel Sauerstoff, oder einer Vakuum-Quelle, die ein Verbindungsbehältnis (1) umfaßt, das dauernd an die Quelle angeschlossen werden kann, und ein Verbindungselement (3), das lösbar an das Verbindungsbehältnis gekoppelt und dauernd mit dem Benutzer verbunden werden kann, wobei das Verbindungselement mit dem Verbindungsbehältnis in einer Halteposition und einer Arbeitsposition gekoppelt werden kann, wobei der Benutzer nur in der Arbeitsposition mit der Quelle verbunden ist, und wobei Anzeigemittel (7, 8 ,10) die Position des Verbindungselements (3) im Verbindungsbehältnis (1) anzeigen, **dadurch gekennzeichnet**, daß die Anzeigemittel (7, 8, 10) ein Fenster (10) umfassen, in dem eine Farbanzeige sichtbar ist, wobei die Anzeigemittel einen im Verbindungselement (3) verschiebbar angebrachten Stift (7) aufweisen, wobei der Stift einen farbigen Kopf (8) besitzt, der während der Bewegung von der Halteposition in die Arbeitsposition durch eine Feder (9) vom Fenster (10) weggedrückt und entgegen der Wirkung der Feder auf das Fenster zubewegt wird, und wobei das Fenster (10) dreidimensional geformt ist, und der Kopf (8) des Stiftes (7) vollständig innerhalb des dreidimensionalen Fensters in der Arbeitsposition liegt.

2. Vorrichtung nach Anspruch 1, **dadurch gekennzeichnet**, daß der Stift (7) mit seinem dem Kopf (8) entgegengesetzen Ende aus dem Verbindungselement (3) vorspringt und mit diesem Ende mit dem Verbindungsbehältnis (1) zusammenwirkt.

3. Vorrichtung nach Anspruch 1, **dadurch gekennzeichnet**, daß die Anzeigemittel (7, 8, 10) im Verbindungselement (3) vorgesehen sind.

4. Vorrichtung nach Anspruch 1 oder 3, **dadurch gekennzeichnet**, daß die Anzeigemittel (7, 8, 10) durch die Bewegung des Verbindungselementes (3) von der Halteposition in die Arbeitsposition angesprochen werden.

5. Vorrichtung nach Anspruch 1 oder 2, **dadurch gekennzeichnet**, daß die Anzeigemittel (7, 8, 10) durch den Gasdruck angesprochen werden.

## Revendications

1. Dispositif pour raccorder un utilisateur à une source d'un gaz médical, comme par exemple de l'oxygène, ou à une source de vide, comprenant une boîte de raccordement (1) qui peut être raccordée de manière permanente à la source, et un organe de raccordement (3) qui peut être accouplé de manière libérable avec la boîte de raccordement et peut être raccordé de manière permanente avec l'utilisateur, dans lequel l'organe de raccordement peut être accouplé avec la boîte de raccordement dans une position de repos et dans une position active, dans lequel l'utilisateur est raccordé à la source uniquement dans la position active et dans lequel un moyen d'indication (7, 8, 10) indique la position de l'organe de raccordement (3) dans la boîte de raccordement (1), caractérisé en ce que le moyen d'indication comprend une fenêtre (10) dans laquelle une indication de couleur est visible, lequel moyen d'indication comprend une tige (7) montée de manière coulissante dans l'organe de raccordement (3), ladite tige ayant une tête colorée (8) éloignée de la fenêtre (10) par poussée d'un ressort (9) et déplacée vers la fenêtre contre l'action du ressort pendant le mouvement depuis la position de repos vers la position active, et en ce que la fenêtre (10) a une forme tridimensionnelle, la tête (8) de la tige (7) se trouvant complètement dans la fenêtre tridimensionnelle dans la position active.

2. Dispositif selon la revendication 1, caractérisé en ce que la tige (7) s'étend, avec son extrémité opposée à la tête (8), à l'extérieur de l'organe de raccordement (3) et coopère avec ladite extrémité avec la boîte de raccordement (1).

3. Dispositif selon la revendication 1, caractérisé en ce que le moyen d'indication (7, 8, 10) est prévu dans l'organe de raccordement (3).

4. Dispositif selon la revendication 1 ou 3, caractérisé en ce que le moyen d'indication (7, 8, 10) est actionné par le mouvement de l'organe de raccordement (3) depuis la position de repos vers la position active.

5. Dispositif selon la revendication 1 ou 2, caractérisé en ce que le moyen d'indication (7, 8, 10) est actionné par la pression de gaz.
